# EUROPEAN PATENT SPECIFICATION

(11) **EP 2 386 274 B1**
(45) Date of publication and mention of the grant of the patent: **10.01.2018**
(21) Application number: 11166287.0
(22) Date of filing: 16.05.2011
(51) Int. Cl.: A61F 2/30, A61F 2/46, A61F 2/28, A61F 2/44

(54) **Interbody spinal implant having internally textured surfaces**
Zwischenwirbelimplantat mit intern texturierten Oberflächen
Implant spinal intervertébral doté de surfaces texturées internes

(30) Priority: 14.05.2010 US 334853 P
(43) Date of publication of application: 16.11.2011
(73) Proprietor: Titan Spine, LLC, Mequon WI 53092 (US)
(72) Inventor: Ullrich, Jr, Peter F., Neenah, WI 54956 (US); Gemas, Kevin W., Thiensville, WI 53092 (US); Patterson, Chad J., Port Washington, WI 53074 (US); Schneider, Jennifer M., Germantown, WI 53022 (US)
(74) Representative: Moore, Michael Richard

(56) References cited:
- WO-A1-98/01091
- WO-A2-2006/119088
- WO-A2-2006/121795
- US-A- 5 865 845
- US-A1- 2005 060 034
- US-A1- 2008 262 623

## Description

### TECHNICAL FIELD

The present invention relates generally to interbody spinal implants and methods of using such implants and, more particularly, to a surface treatment for spinal implants that promotes bone ingrowth once the implant is positioned within a patient's spinal column.

### BACKGROUND OF THE INVENTION

In the simplest terms, the spine is a column made of vertebrae and discs. The vertebrae provide the support and structure of the spine while the spinal discs, located between the vertebrae, act as cushions or "shock absorbers." These discs also contribute to the flexibility and motion of the spinal column. Over time, the discs may become diseased or infected, may develop deformities such as tears or cracks, or may simply lose structural integrity (e.g., the discs may bulge or flatten). Impaired discs can affect the anatomical functions of the vertebrae, due to the resultant lack of proper biomechanical support, and are often associated with chronic back pain.

Several surgical techniques have been developed to address spinal defects, such as disc degeneration and deformity. Spinal fusion has become a recognized surgical procedure for mitigating back pain by restoring biomechanical and anatomical integrity to the spine. Spinal fusion techniques involve the removal, or partial removal, of at least one intervertebral disc and preparation of the disc space for receiving an implant by shaping the exposed vertebral endplates. An implant is then inserted between the opposing endplates.

Spinal fusion procedures can be achieved using a posterior or an anterior approach. Anterior interbody fusion procedures generally have the advantages of reduced operative times and reduced blood loss. Further, anterior procedures do not interfere with the posterior anatomic structure of the lumbar spine. Anterior procedures also minimize scarring within the spinal canal while still achieving improved fusion rates, which is advantageous from a structural and biomechanical perspective. These generally preferred anterior procedures are particularly advantageous in providing improved access to the disc space, and thus correspondingly better endplate preparation.

Several interbody implant systems have been introduced to facilitate interbody fusion. Traditional threaded implants involve at least two cylindrical bodies, each typically packed with bone graft material, surgically placed on opposite sides of the mid-sagittal plane through pre-tapped holes within the intervertebral disc space. This location is not the preferable seating position for an implant system, however, because only a relatively small portion of the vertebral endplate is contacted by these cylindrical implants. Accordingly, these implant bodies will likely contact the softer cancellous bone rather than the stronger cortical bone, or apophyseal rim, of the vertebral endplate. The seating of these threaded cylindrical implants may also compromise biomechanical integrity by reducing the area in which to distribute mechanical forces, thus increasing the apparent stress experienced by both the implant and vertebrae. Still further, a substantial risk of implant subsidence (defined as sinking or settling) into the softer cancellous bone of the vertebral body may arise from such improper seating.

In contrast, open ring-shaped cage implant systems are generally shaped to mimic the anatomical contour of the vertebral body. Traditional ring-shaped cages are generally comprised of allograft bone material, however, harvested from the human femur. Such allograft bone material restricts the usable size and shape of the resultant implant. For example, many of these femoral ring-shaped cages generally have a medial-lateral width of less than 25 mm. Therefore, these cages may not be of a sufficient size to contact the strong cortical bone, or apophyseal rim, of the vertebral endplate. These size-limited implant systems may also poorly accommodate related instrumentation such as drivers, reamers, distractors, and the like. For example, these implant systems may lack sufficient structural integrity to withstand repeated impact and may fracture during implantation. Still further, other traditional non-allograft ring-shaped cage systems may be size-limited due to varied and complex supplemental implant instrumentation which may obstruct the disc space while requiring greater exposure of the operating space. These supplemental implant instrumentation systems also generally increase the instrument load upon the surgeon.

The surgical procedure corresponding to an implant system should preserve as much vertebral endplate bone surface as possible by minimizing the amount of bone removed. This vertebral endplate bone surface, or subchondral bone, is generally much stronger than the underlying cancellous bone. Preservation of the endplate bone stock ensures biomechanical integrity of the endplates and minimizes the risk of implant subsidence. Thus, proper interbody implant design should provide for optimal seating of the implant while utilizing the maximum amount of available supporting vertebral bone stock.

Traditional interbody spinal implants generally do not seat properly on the preferred structural bone located near the apophyseal rim of the vertebral body, which is primarily composed of preferred dense subchondral bone. To address this problem, the art is replete with surface geometries and textures to promote proper seating of the implant. For example, current spinal interbody implant designs have bone apposition surfaces that are contoured or shaped to enhance retention in the intradiscal space when placed between two vertebrae. The top and bottom surfaces may have texturing or features to improve load transferring surface area, engage bone structures and resist movement under loads imparted by patient activity. In addition to large features and shapes, the surfaces may also have microscopic features and shapes intended to aid in the biologic attachment of the vertebrae by biologically interacting with the bone cells. Most of these implants also have internal passages for the placement of graft and bone growth enhancing materials inside of the implant intended to aid in the formation of a stable fusion. This graft material forms part of the fusion that is intended to form with the disc space of the patient by providing biologically compatible material, often including patient-derived or synthesized biologic materials. Graft materials are intended to be remodeled and/or absorbed during the healing phases and the stability of the implant and graft materials is critical to the successful formation of new bone tissues and long term fusion stability.

WO2006/121795 and US2008/262623 relate to interbody spinal implants and methods of using such implants. The implants may be substantially hollow and have a generally oval-shaped transverse cross-sectional area. A roughened surface topography may be included on at least a portion of the top and bottom outer surfaces of the implant for gripping adjacent bone and inhibiting migration of the implant. WO 2006/119088 describes a motion-restoring prosthesis and an invertebral spacer. The bone-buttressing surfaces may be roughened or made uneven and coated with thin micron coating of a bone-on growth attachment promoting material for enhancing the affixation of the associated bone facing surface to the implant. US2005/060034 relates to a modular prosthetic device and an anchoring device for modular prosthetic devices. The anchoring device is intended to be inserted within an inter-vertebral space, and comprises a vertebral-engaging member that may be coated with a bone-growth promoting substance to enhance adhesion of bone to the device. The vertebral-engaging member may have a roughened surface prior to coating with the substance.

US 5 865 845 A discloses a spinal disc implant being made of titanium or an alloy thereof, said implant comprises a body with a central opening defined by an interior surface. The interior surface has a series of circumferential scorings to help hold by friction any material, e.g. a synthetic bone material, inserted in the central opening. One problem with such prior art implants, however, is that they do not promote bone growth into the graft material in the internal passage of the implant. Accordingly, there is a need in the art for interbody spinal implants that better utilize the graft materials and promotes bone growth in the internal passages of the implants.

### BRIEF SUMMARY OF THE INVENTION

In accordance with the present invention, there is provided an interbody spinal implant as defined in claim 1.

Further advantages are achieved by the embodiments indicated by the dependent claims.

A method of forming a surface roughness on an interior wall surface of an interbody spinal implant is described. The method comprises the steps of: providing an interbody spinal implant including a body, the body comprising: a top surface; a bottom surface; opposing lateral sides; and opposing anterior and posterior portions; the top surface, bottom surface, opposing lateral sides, internal wall surface, and opposing anterior and posterior portions defining a substantially hollow center having a single vertical aperture defining an interior wall surface, the single vertical aperture (a) extending from the top surface to the bottom surface, (b) having a size and shape predetermined to maximize the surface area of the top surface and the bottom surface available proximate the anterior and posterior portions while maximizing both radiographic visualization and access to the substantially hollow center, and (c) defining a transverse rim; and is characterized in this aspect by etching at least a portion of the interior wall surface such that at least a portion of the internal wall surface has a roughened surface topography.

It should be appreciated that any feature or features described herein in relation to one aspect of the invention can be incorporporated into any other aspect of the invention, unless it is otherwise apparent that such incorporation would not be intended.

### BRIEF DESCRIPTION OF THE DRAWING

The invention is best understood from the following detailed description when read in connection with the accompanying drawing. It is emphasized that, according to common practice, the various features of the drawing are not to scale. On the contrary, the dimensions of the various features are arbitrarily expanded or reduced for clarity. Included in the drawing are the following figures:
FIG. 1 shows a perspective view of a first embodiment of the interbody spinal implant having a generally oval shape and roughened surface topography on the top surface;
FIG. 2 depicts a top view of the first embodiment of the interbody spinal implant;
FIG. 3 depicts an anterior view of the first embodiment of the interbody spinal implant; and
FIG. 4 depicts a posterior view of the first embodiment of the interbody spinal implant;
FIG. 5 shows a confocal laser microscopy image of an comparative polyetheretherketone (PEEK) surface;
FIG. 6 shows a confocal laser microscopy image of a comparative smooth titanium alloy (sTi or sTiAIV) surface;
FIG. 7 shows a confocal laser microscopy image of an exemplary rough titanium alloy (rTi or rTiAIV) surface;
FIG. 8 shows SEM images of the PEEK surface of FIG. 5 at 1K x and 2K x magnification;
FIG. 9 shows SEM images of the smooth titanium alloy surface of FIG. 6 at 1K x and 2K x magnification;
FIG. 10 shows SEM images of the rough titanium alloy surface of FIG. 7 at 1K x and 2K x magnification;
FIG. 11 shows a graph of the cell number for human MG63 osteoblast-like cells cultured on tissue culture polystyrene (TCPS), PEEK, sTI, and rTi surfaces.
FIG. 12 shows a graph of alkaline phosphatase specific activity for human MG63 osteoblast-like cells cultured on tissue culture polystyrene (TCPS), PEEK, sTI, and rTi surfaces.
FIG. 13 shows a graph of osteocalcin levels for human MG63 osteoblast-like cells cultured on tissue culture polystyrene (TCPS), PEEK, sTI, and rTi surfaces.
FIG. 14 shows a graph of osteoprotegerin levels for human MG63 osteoblast-like cells cultured on tissue culture polystyrene (TCPS), PEEK, sTI, and rTi surfaces.
FIG. 15 shows a graph of latent TGF-β1 levels for human MG63 osteoblast-like cells cultured on tissue culture polystyrene (TCPS), PEEK, sTI, and rTi surfaces.
FIG. 16 shows a graph of active TGF-β1 levels for human MG63 osteoblast-like cells cultured on tissue culture polystyrene (TCPS), PEEK, sTI, and rTi surfaces.
FIG. 17 shows a graph of BMP2 levels for human MG63 osteoblast-like cells cultured on tissue culture polystyrene (TCPS), PEEK, sTI, and rTi surfaces.
FIG. 18 shows a graph of active BMP4 levels for human MG63 osteoblast-like cells cultured on tissue culture polystyrene (TCPS), PEEK, sTI, and rTi surfaces.
FIG. 19 shows a graph of active BMP7 levels for human MG63 osteoblast-like cells cultured on tissue culture polystyrene (TCPS), PEEK, sTI, and rTi surfaces.
FIG. 20 shows a graph of ITGA1 expression for human MG63 osteoblast-like cells cultured on tissue culture polystyrene (TCPS), PEEK, sTI, and rTi surfaces.
FIG. 21 shows a graph of ITGA2 expression for human MG63 osteoblast-like cells cultured on tissue culture polystyrene (TCPS), PEEK, sTI, and rTi surfaces.
FIG. 22 shows a graph of ITGAV expression for human MG63 osteoblast-like cells cultured on tissue culture polystyrene (TCPS), PEEK, sTI, and rTi surfaces.
FIG. 23 shows a graph of ITGB1 expression for human MG63 osteoblast-like cells cultured on tissue culture polystyrene (TCPS), PEEK, sTI, and rTi surfaces.
FIG. 24 shows a graph of BMP2 expression for human MG63 osteoblast-like cells cultured on tissue culture polystyrene (TCPS), PEEK, sTI, and rTi surfaces.
FIG. 25 shows a graph of BMP4 expression for human MG63 osteoblast-like cells cultured on tissue culture polystyrene (TCPS), PEEK, sTI, and rTi surfaces.
FIG. 26 shows a graph of NOG expression for human MG63 osteoblast-like cells cultured on tissue culture polystyrene (TCPS), PEEK, sTI, and rTi surfaces.
FIG. 27 shows a graph of GREM1 expression for human MG63 osteoblast-like cells cultured on tissue culture polystyrene (TCPS), PEEK, sTI, and rTi surfaces.

### DETAILED DESCRIPTION OF THE INVENTION

Certain embodiments of the invention may be especially suited for placement between adjacent human vertebral bodies. The implants of the invention may be used in procedures such as Anterior Lumbar Interbody Fusion (ALIF), Posterior Lumbar Interbody Fusion (PLIF), Transforaminal Lumbar Interbody Fusion (TLIF), and cervical fusion. Certain embodiments do not extend beyond the outer dimensions of the vertebral bodies.

The ability to achieve spinal fusion is directly related to the available vascular contact area over which fusion is desired, the quality and quantity of the fusion mass, and the stability of the interbody spinal implant. Interbody spinal implants, as now taught, allow for improved seating over the apophyseal rim of the vertebral body. Still further, interbody spinal implants, as now taught, better utilize this vital surface area over which fusion may occur and may better bear the considerable biomechanical loads presented through the spinal column with minimal interference with other anatomical or neurological spinal structures. Even further, interbody spinal implants allow for improved visualization of implant seating and fusion assessment. Interbody spinal implants, as now taught, also facilitate osteointegration with the surrounding living bone.

Anterior interbody spinal implants can be preferably made of a durable material such as stainless steel, stainless steel alloy, titanium, or titanium alloy, but can also be made of other durable materials such as, but not limited to, polymeric, ceramic, and composite materials. For example, certain examples may be comprised of a biocompatible, polymeric matrix reinforced with bioactive fillers, fibers, or both. Certain examples may be comprised of urethane dimethacrylate (DUDMA)/tri-ethylene glycol dimethacrylate (TEDGMA) blended resin and a plurality of fillers and fibers including bioactive fillers and E-glass fibers. Durable materials may also consist of any number of pure metals, metal alloys, or both. Titanium and its alloys are the materials which are used for the interbody spinal implant according to the invention due to their acceptable, and desirable, strength and biocompatibility. In this manner, certain examples of the interbody spinal implant may have improved structural integrity and may better resist fracture during implantation by impact. Interbody spinal implants, as now taught, may therefore be used as a distractor during implantation.

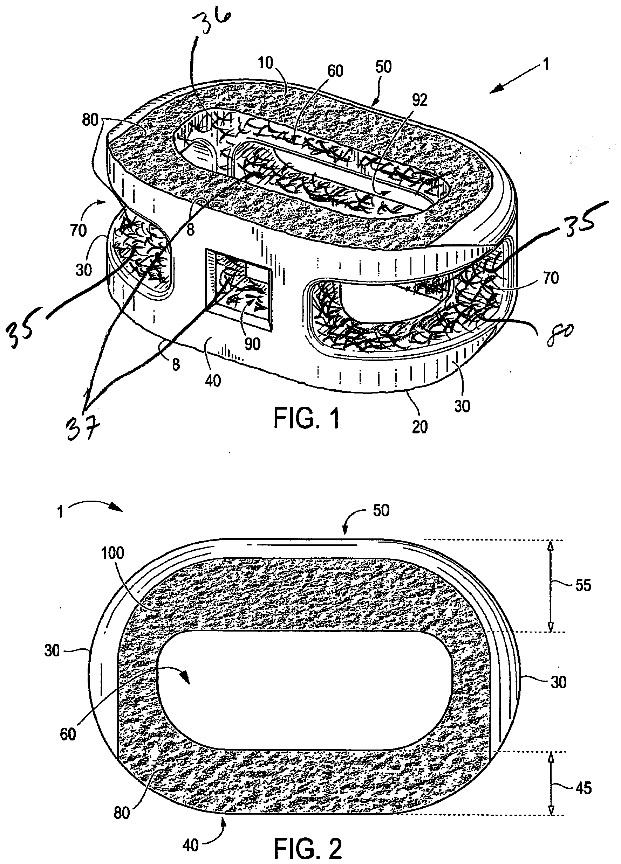

Referring now to the drawings, in which like reference numbers refer to like elements throughout the various figures that comprise the drawings, FIGS. 1-4 show perspective views of a first embodiment of the interbody spinal implant 1 especially well adapted for use in an ALIF procedure. The interbody spinal implant 1 includes a body having a top surface 10, a bottom surface 20, opposing lateral sides 30, opposing anterior 40 and posterior 50 portions, internal medial to lateral surfaces 35, and internal anterior to posterior passage surface 37. As shown in FIGS. 1-4, top surface 10, bottom surface 20, the internal medial to lateral surfaces 35, and the internal anterior to posterior passage surface have a roughened topography 80. A roughened topography 80 on the top surface 10 and bottom surface 20 as shown in FIGS. 1-4 is preferable but is not a necessary component of the invention.

Certain examples of the interbody spinal implant 1 are substantially hollow and have a generally oval-shaped transverse cross-sectional area with smooth, rounded, or both smooth and rounded lateral sides and posterior-lateral corners. As used in this document, "substantially hollow" means at least about 33% of the interior volume of the interbody spinal implant 1 is vacant. For example, the volume defined by the body of the implant may be at least 33% void, and in some examples the void may be at least 40%, at least 50% or at least 60% of the internal volume of the implant. The implant 1 includes at least one vertical aperture 60 defined by internal walls 36 that extends the entire height of the implant body. Internal walls 36 have a roughened topography 80. As illustrated in the top view of FIG. 2, the vertical aperture 60 further defines a transverse rim 100 having a greater posterior portion thickness 55 than an anterior portion thickness 45.

In at least one example, the opposing lateral sides 30 and the anterior portion 40 have a rim thickness of about 5 mm, while the posterior portion 50 has a rim thickness of about 7 mm. However, the rim thickness of the anterior portion 40 may be any thickness, such as 2-8 mm, and suitably including 3-7 mm, or larger or smaller; and the rim thickness of the posterior portion 50 may be of any thickness, such as 4-10 mm, and suitably including 5-9 mm, or larger or smaller. Thus, the rim posterior portion thickness 55 may allow for better stress sharing between the implant 1 and the adjacent vertebral endplates and helps to compensate for the weaker posterior endplate bone. In certain examples, the transverse rim 100 has a generally large surface area and contacts the vertebral endplate. The transverse rim 100 may act to better distribute contact stresses upon the implant 1, and hence minimize the risk of subsidence while maximizing contact with the apophyseal supportive bone. It is also possible for the transverse rim 100 to have a substantially constant thickness (i.e., for the anterior portion thickness 45 to be substantially the same as the posterior portion thickness 55) or, in fact, for the posterior portion 50 to have a rim thickness less than that of the opposing lateral sides 30 and the anterior portion 40. Some studies have challenged the characterization of the posterior endplate bone as weaker.

It is generally believed that the surface of an implant determines its ultimate ability to integrate into the surrounding living bone. Without being limited by theory, it is hypothesized that the cumulative effects of at least implant composition, implant surface energy, and implant surface roughness play a major role in the biological response to, and osteointegration of, an implant device. Thus, implant fixation may depend, at least in part, on the attachment and proliferation of osteoblasts and like-functioning cells upon the implant surface. Still further, it appears that these cells attach more readily to relatively rough surfaces rather than smooth surfaces. In this manner, a surface may be bioactive due to its ability to facilitate cellular attachment and osteointegration.

Accordingly, the surface roughened topography 80 on the internal surfaces of the implant according to the invention, including internal walls 36, internal medial to lateral surfaces 35, and internal anterior to posterior passage surface 37, better promotes the osteointegration of certain embodiments of the invention. The surface roughened topography 80 on top surface 10 and bottom surface 20 further provide better grip the vertebral endplate surfaces and inhibit implant migration upon placement and seating. The average roughness of the roughened surface topography should be greater than 90 nm. In some examples, the roughened surface topography is in the range of bout 0.1 to 5 µm, from about 0.5 to 4 µm, or from about 1 to 3 µm. In a preferred example, the roughened surface topography has an average roughness of about 1.30 to 2.32 µm.

Interbody spinal implants in accordance with preferred embodiments of the invention (which includes the internal surface roughened topography), have, in combination, a macro average surface roughness of greater than 100 µm (height from peak to valley), a microtexture average surface roughness of from about 0.5 to about 100 µm (height from peak to valley) and a nanotexture average surface roughness of from about 1 to about 1000 nm (height from peak to valley). Surface roughness may be measured using a laser profilometer or other standard instrumentation.

The roughened topography 80 may be obtained through a variety of techniques including, without limitation, chemical etching, shot peening, plasma etching, laser etching, or abrasive blasting (such as sand or grit blasting). The interbody spinal implant 1 is comprised of titanium, or a titanium alloy, having the surface roughened topography 80. The surfaces of the implant 1 are preferably bioactive.

In a preferred embodiment of the invention, the roughened topography 80 is obtained via the repetitive masking and chemical or electrochemical milling processes described in U.S. Patent Nos. 5,258,098; 5,507,815; 5,922,029; and 6,193,762. Where chemical etching is employed, the surface is prepared through an etching process that utilizes the random application of a maskant and subsequent etching of the metallic substrate in areas unprotected by the maskant. This etching process is repeated a number of times as necessitated by the amount and nature of the irregularities required for any particular application. Control of the strength of the etchant material, the temperature at which the etching process takes place, and the time allotted for the etching process allow fine control over the resulting surface produced by the process. The number of repetitions of the etching process can also be used to control the surface features.

Interbody spinal implants in accordance with the invention are comprised of titanium, or a titanium alloy. By way of example, an etchant mixture of nitric acid (HNO₃) and hydrofluoric (HF) acid may be repeatedly applied to a titanium surface to produce an average etch depth of about 0.53 mm. In another example, chemical modification of the titanium implant surfaces can be achieved using HF and a combination of hydrochloric acid and sulfuric acid (HCl/H₂SO₄). In a dual acid etching process, the first exposure is to HF and the second is to HCl/H₂SO₄. Chemical acid etching alone of the titanium implant surface has the potential to greatly enhance osteointegration without adding particulate matter (e.g., hydroxyapatite) or embedding surface contaminants (e.g., grit particles).

Accordingly, an example includes a method of forming a surface roughness on an interior wall surface of an interbody spinal implant, the method comprising the steps of: providing an interbody spinal implant including a body, the body comprising: a top surface; a bottom surface; opposing lateral sides; and opposing anterior and posterior portions; the top surface, bottom surface, opposing lateral sides, internal wall surface, and opposing anterior and posterior portions defining a substantially hollow center having a single vertical aperture defining an interior wall surface, the single vertical aperture (a) extending from the top surface to the bottom surface, (b) having a size and shape predetermined to maximize the surface area of the top surface and the bottom surface available proximate the anterior and posterior portions while maximizing both radiographic visualization and access to the substantially hollow center, and (c) defining a transverse rim; and etching at least a portion of the interior wall surface such that at least a portion of the internal wall surface has a roughened surface topography.

Certain examples of the implant 1 are generally shaped to reduce the risk of subsidence, and improve stability, by maximizing contact with the apophyseal rim of the vertebral endplates. Examples may be provided in a variety of anatomical footprints having a medial-lateral width ranging from about 32 mm to about 44 mm. Interbody spinal implants, as now taught, generally do not require extensive supplemental or obstructive implant instrumentation to maintain the prepared disc space during implantation. Thus, the interbody spinal implant 1 and associated implantation methods, according to examples, allow for larger sized implants as compared with the size-limited interbody spinal implants known in the art. This advantage allows for greater medial-lateral width and correspondingly greater contact with the apophyseal rim.

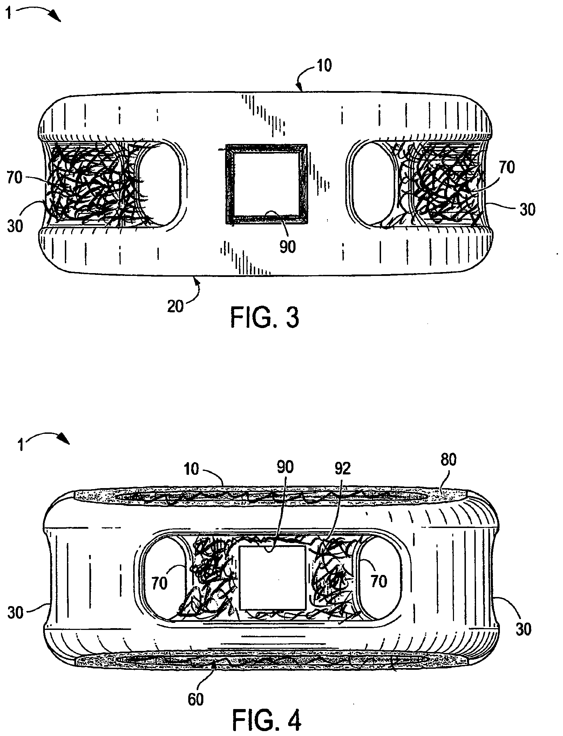

FIG. 3 depicts an anterior view, and FIG. 4 depicts a posterior view, of an embodiment of the interbody spinal implant 1. As illustrated in FIGS. 1 and 3, the implant 1 has an opening 90 in the anterior portion 40. As illustrated in FIGS. 3 and 4, in one example, the posterior portion 50 has a similarly shaped opening 90. In another example, as illustrated in FIG. 1, only the anterior portion 40 has the opening 90 while the posterior portion 50 has an alternative opening 92 (which may have a size and shape different from the opening 90).

The opening 90 has a number of functions. One function is to facilitate manipulation of the implant 1 by the caretaker. Thus, the caretaker may insert a surgical tool into the opening 90 and, through the engagement between the surgical tool and the opening 90, manipulate the implant 1. The opening 90 may be threaded to enhance the engagement.

The implant 1 may further include at least one transverse aperture 70 that extends the entire transverse length of the implant body. These transverse apertures 70 may provide improved visibility of the implant 1 during surgical procedures to ensure proper implant placement and seating, and may also improve post-operative assessment of implant fusion. Still further, the substantially hollow area defined by the implant 1 may be filled with cancellous autograft bone, allograft bone, DBM, porous synthetic bone graft substitute, BMP, or combinations of these materials (collectively, bone graft materials), to facilitate the formation of a solid fusion column within the spine of a patient. Roughened topography of, for example, internal wall surface 36, promote osteointegration through the space of the implant occupied such material which ultimately improves the stability of the implant.

The anterior portion 40, or trailing edge, of the implant 1 is preferably generally greater in height than the opposing posterior portion 50. Accordingly, the implant 1 may have a lordotic angle to facilitate sagittal alignment. The implant 1 may better compensate, therefore, for the generally less supportive bone found in the posterior regions of the vertebral endplate. The posterior portion 50 of the interbody implant 1, preferably including the posterior-lateral corners, may also be highly radiused, thus allowing for ease of implantation into the disc space. Thus, the posterior portion 50 may have a generally blunt nosed profile. The anterior portion 40 of the implant 1 may also preferably be configured to engage a delivery device, driver, or other surgical tool (and, therefore, may have an opening 90).

As illustrated in FIG. 1, the anterior portion 40 of the implant 1 is substantially flat. Thus, the anterior portion 40 provides a face that can receive impact from a tool, such as a surgical hammer, to force the implant 1 into position. The implant 1 has a sharp edge 8 where the anterior portion 40 meets the top surface 10, where the anterior portion 40 meets the bottom surface 20, or in both locations. The sharp edge or edges 8 function to resist pullout of the implant 1 once it is inserted into position.

Certain examples are particularly suited for use during interbody spinal implant procedures (or vertebral body replacement procedures) and may act as a final distractor during implantation, thus minimizing the instrument load upon the surgeon. For example, in such a surgical procedure, the spine may first be exposed via an anterior approach and the center of the disc space identified. The disc space is then initially prepared for implant insertion by removing vertebral cartilage. Soft tissue and residual cartilage may then also be removed from the vertebral endplates.

Vertebral distraction may be performed using trials of various-sized examples of the interbody spinal implant 1. The determinatively sized interbody implant 1 may then be inserted in the prepared disc space for final placement. The distraction procedure and final insertion may also be performed under fluoroscopic guidance. The substantially hollow area within the implant body may optionally be filled, at least partially, with bone fusion-enabling materials such as, without limitation, cancellous autograft bone, allograft bone, DBM, porous synthetic bone graft substitute, BMP, or combinations of those materials. Such bone fusion-enabling material may be delivered to the interior of the interbody spinal implant 1 using a delivery device mated with the opening 90 in the anterior portion 40 of the implant 1. Interbody spinal implants 1, as now taught, are generally larger than those currently known in the art, and therefore have a correspondingly larger hollow area which may deliver larger volumes of fusion-enabling bone graft material. The bone graft material may be delivered such that it fills the full volume, or less than the full volume, of the implant interior and surrounding disc space appropriately.

Although the implant of the invention has been mainly described as an implant that is especially well adapted for use in an ALIF procedure. The roughened topography of the internal surfaces of the implant according to the invention, however, is also applicable to other implants such as, for example, those used better suited for PLIF, TLIF, or cervical fusion procedures as are disclosed in U.S. patent application Publication No. 2008/0262623.

The following examples are presented for illustration.

### EXAMPLES

### Test Method

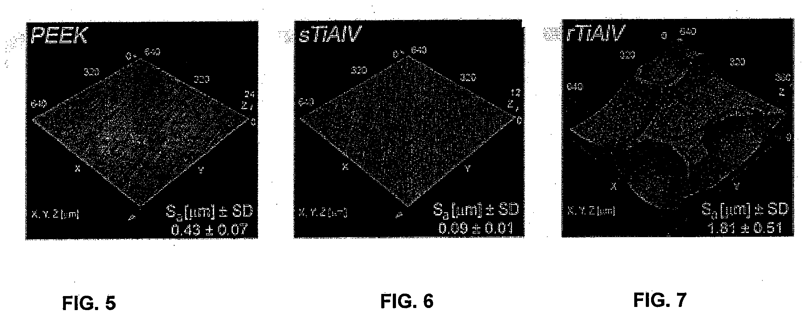

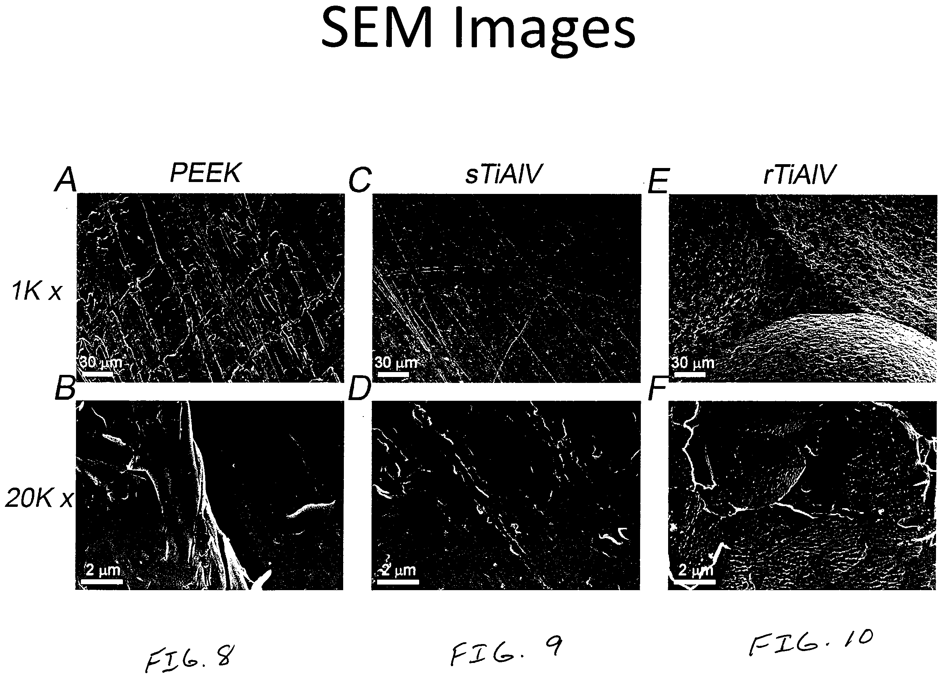

Human osteoblast-like MG63 cells were cultured on tissue culture polystyrene (TCPS), PEEK, or smooth [sTi6AI4V] and rough [rTi6AI4V] surfaces. FIG. 5 shows a confocal laser miscroscopy image of the PEEK surface; FIG. 6 shows a confocal laser miscroscopy image of the sTiAIV surface; and FIG. 7 shows a confocal laser miscroscopy image of the rTiAIV surface. FIG. 8 shows a SEM image of the PEEK surface at 1K x and 20K x magnification; FIG. 9 shows a SEM image of the sTiAIV surface at 1K x and 20K x magnification; FIG. 10 shows a SEM image of the rTiAIV surface at 1K x magnification Gene expression was measured by qPCR. Osteoblast maturation was assessed by analysis of cell number, alkaline phosphatase activity (ALP), and secreted osteocalcin, osteoprotegerin, TGF-β1, BMP2, BMP4, and BMP7. Data are mean ±SEM (n=6/condition), analyzed by ANOVA with Bonferroni's modification of Student's t-test.

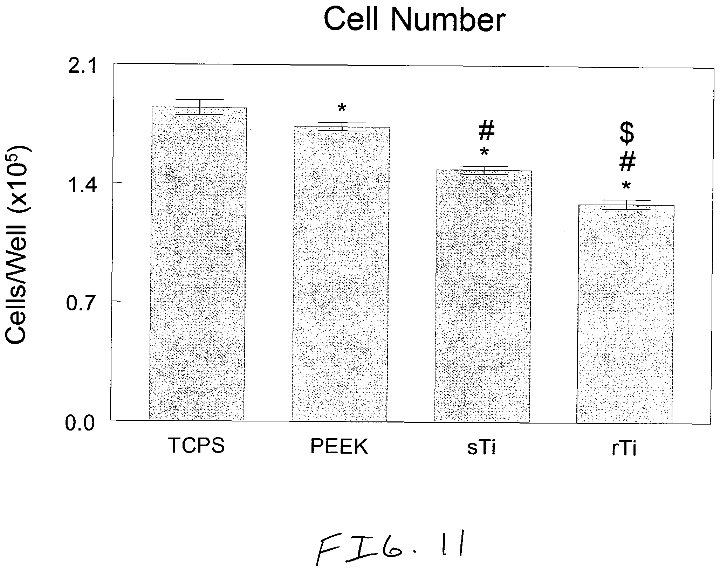

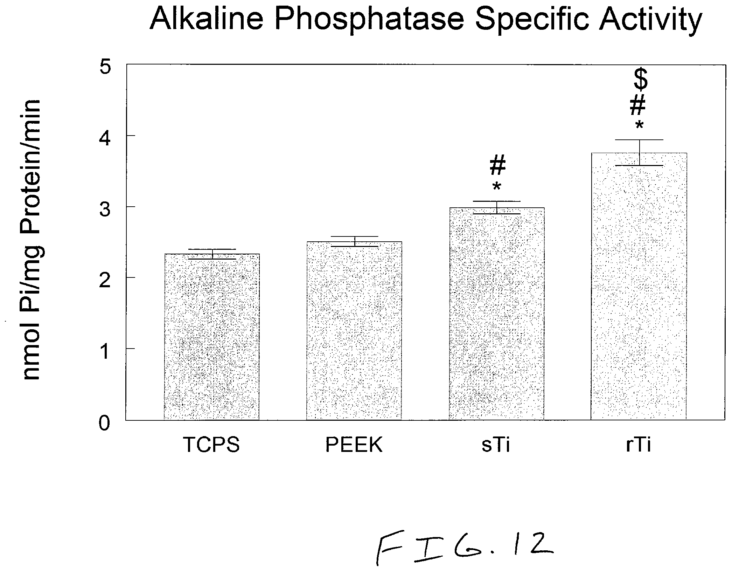

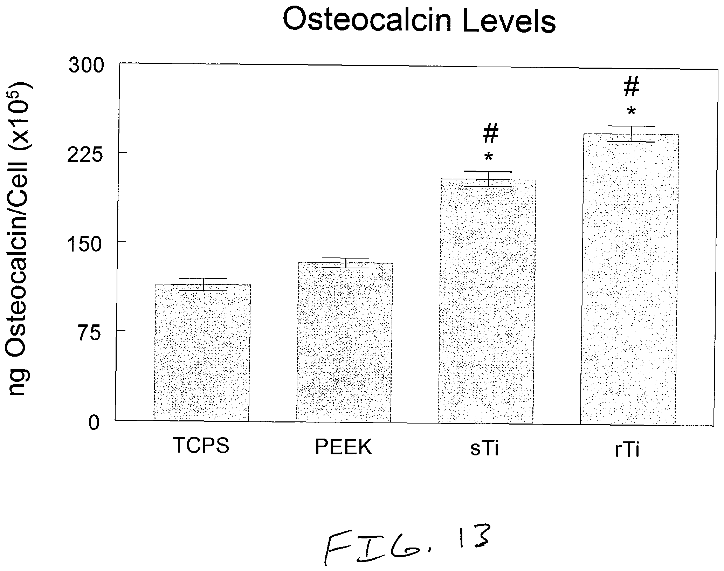

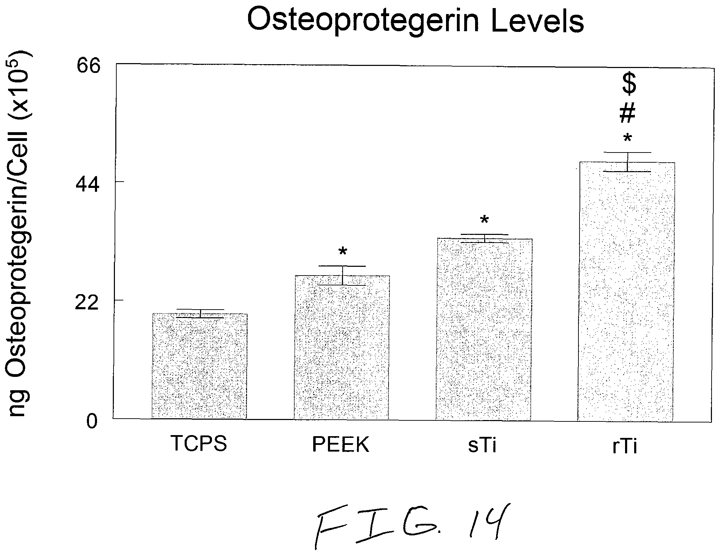

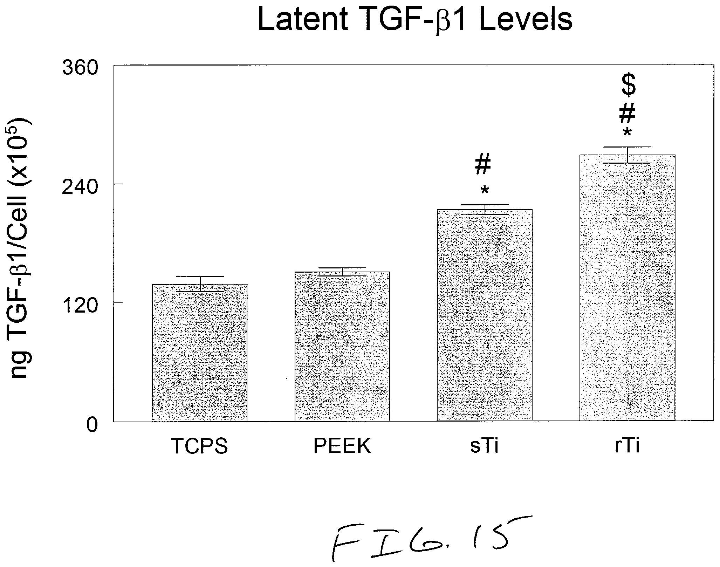

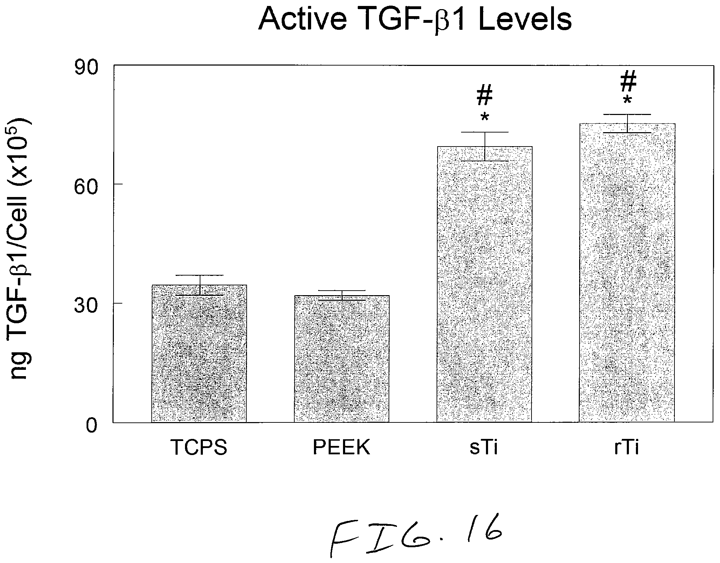

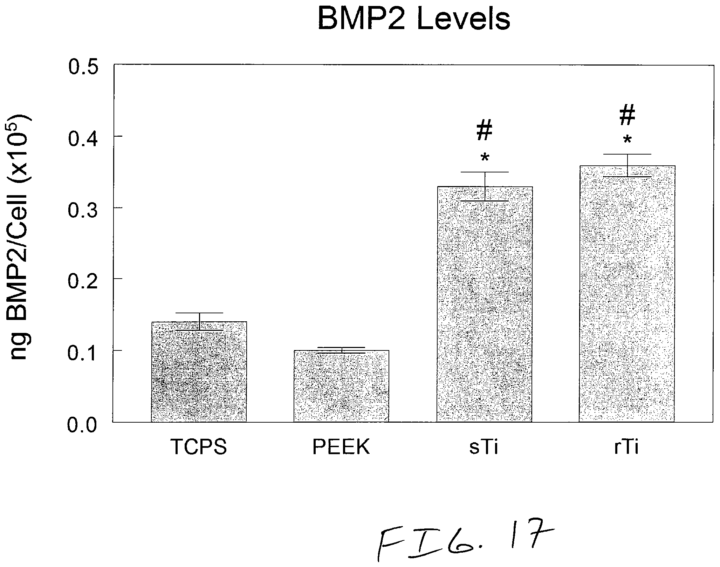

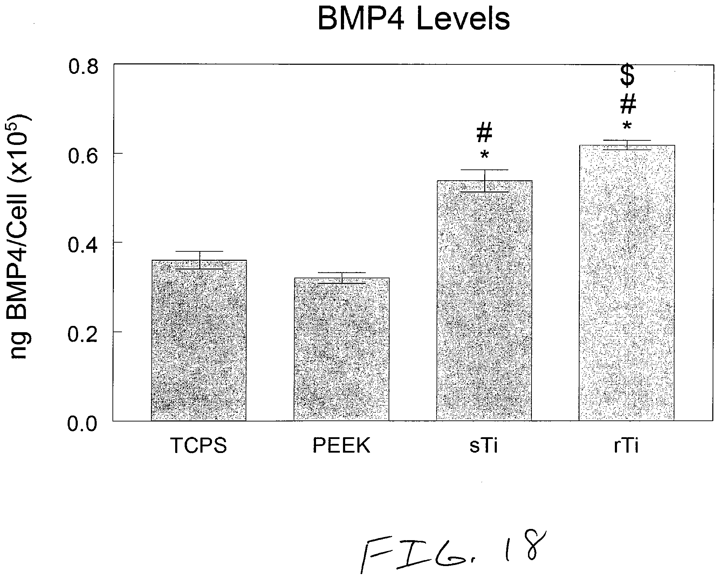

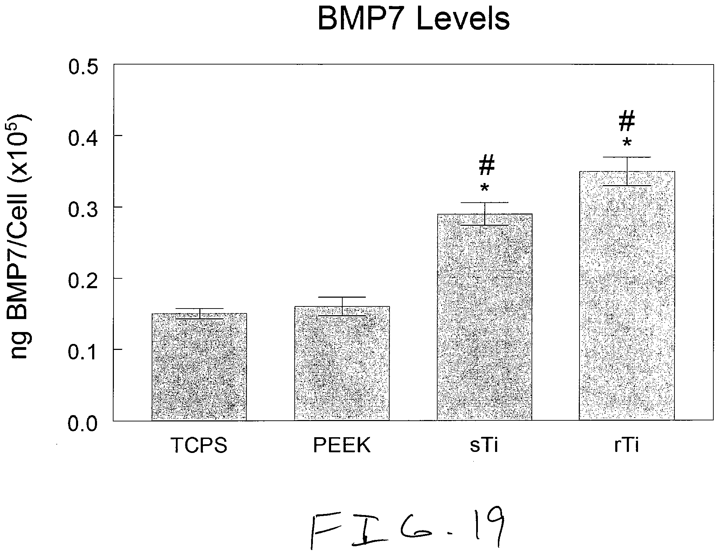

Human MG63 osteoblast-like cells were harvested 24 hours after confluence on TCPS. Cell number, alkaline phosphatase specific activity in cell lysates and levels of osteocalcin, osteoprotegerin, active TGF-β1, latent TGF-β1, BMP2 and BMP4 in the conditioned media were measured. The results of these measurements are shown in FIGS. 11-19, respectively. P-values were as follows: *p<0.05, v. TCPS; #p<0.05, v. PEEK; $p<0.05, v. sTiAIV.

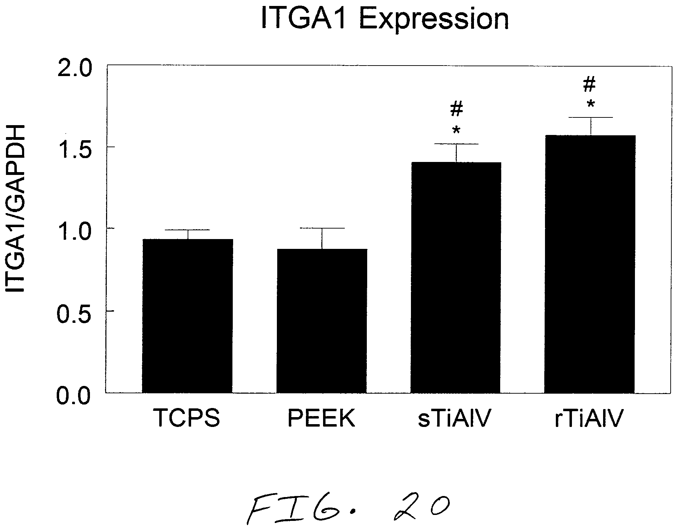

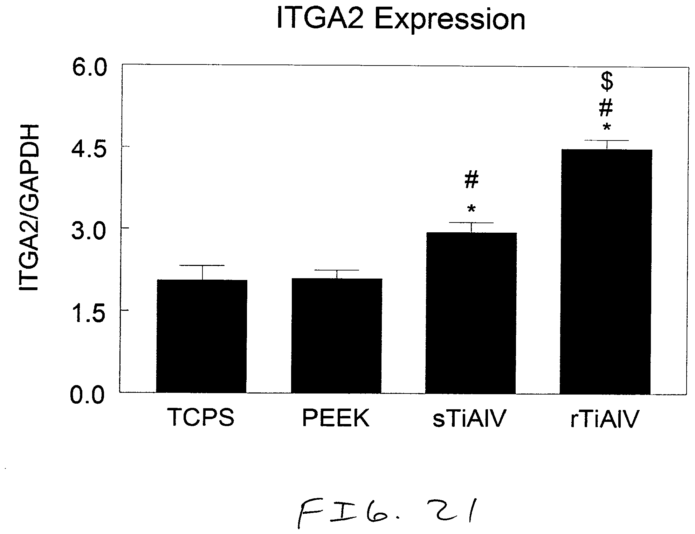

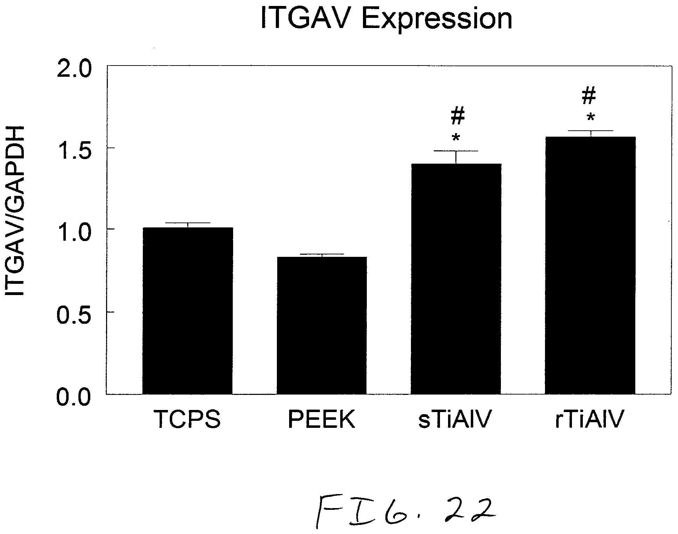

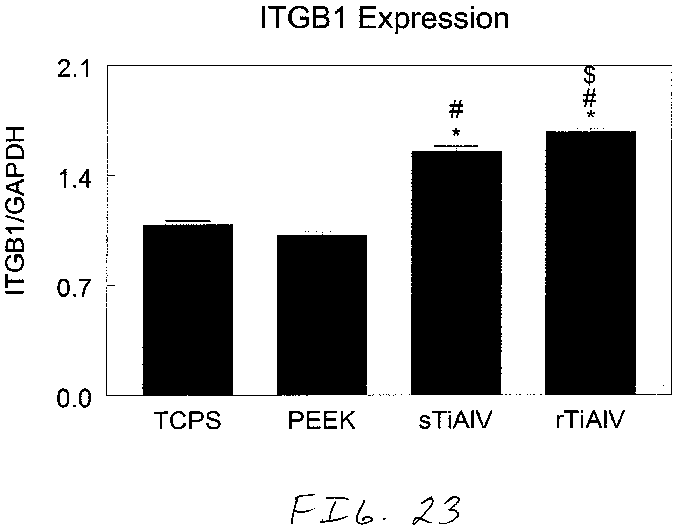

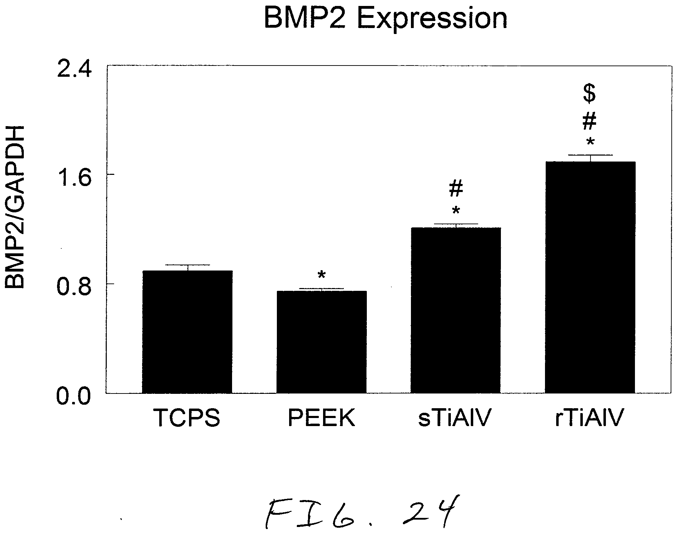

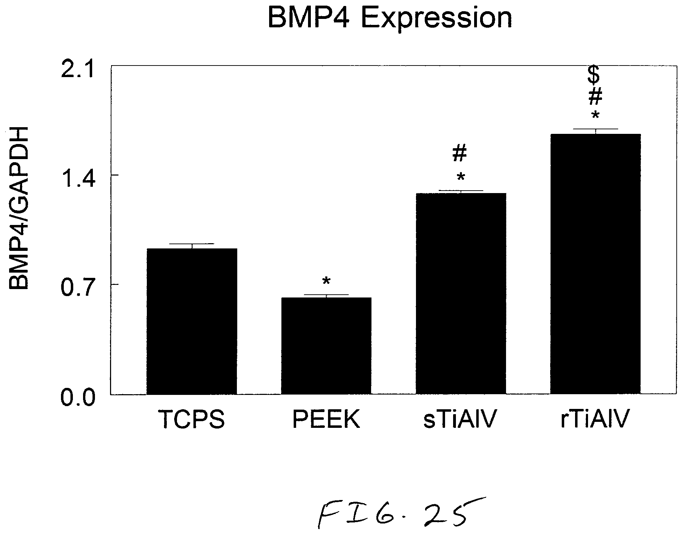

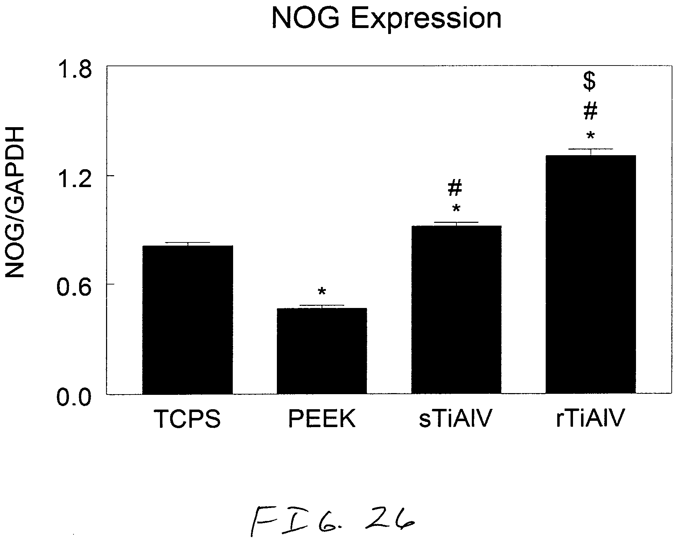

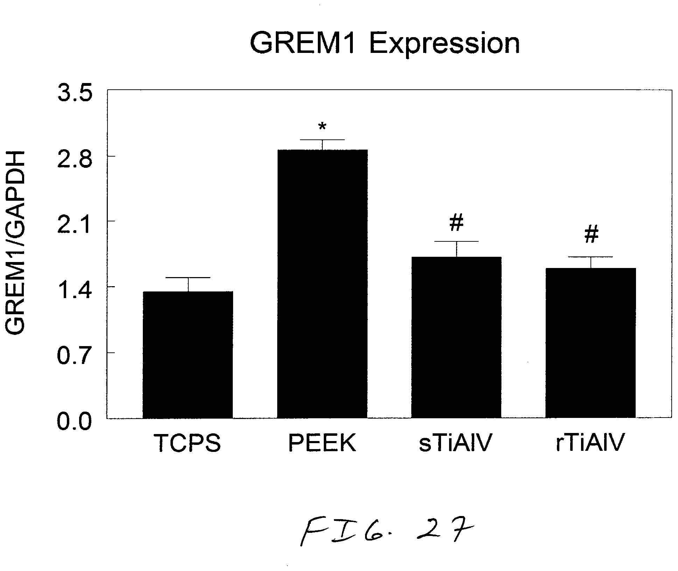

Human MG63 osteoblast-like cells were harvested 12 hours after confluence on TCPS. Levels of mRNA for integrins alpha 1 (ITGA1), alpha 2 (ITGA2), alpha v (ITGAV), and beta 1 (ITGB1), BMP2 (A) and BMP4, and BMP inhibitors noggin (NOG) and gremlin 1 (GREM1) were measured by real-time qPCR and normalized to GAPDH. The results of these measurements are shown in FIGS. 20-27, respectively. P-values were as follows: *p<0.05, v. TCPS; #p<0.05, v. PEEK; $p<0.05, v. sTiAIV.

Results: The results indicated that osteoblasts on Ti6AI4V surfaces present a more mature phenotype than osteoblasts grown on PEEK. Cells on Ti6AI4V, but not PEEK, produced an osteogenic environment. Osteoblasts cultured on Ti6AI4V produced and regulated BMP pathway molecules, increasing BMP2, BMP4, BMP7, and physiologic BMP inhibitors. One reason for the differential responses of osteoblasts to PEEK and TiALV may have been from differences in integrin expression and downstream signaling by these receptors. Taken together, surface properties, including the composition of the bulk material, are important in directing cell response to implant materials, ultimately affecting implant success. The results demonstrated that Ti6AI4V surfaces positively modulate osteoblast maturation and regulated BMP signaling.

The foregoing examples and description of the preferred embodiments should be taken as illustrating, rather than as limiting the present invention as defined by the claims. As will be readily appreciated, numerous variations and combinations of the features set forth above may be utilized without departing from the present invention as set forth in the claims.

## Claims

1. An interbody spinal implant (1) including a titanium or titanium alloy body, the body comprising:
a top surface (10);
a bottom surface (20);
opposing lateral sides (30);
opposing anterior (40) and posterior (50) portions; and
at least one vertical aperture (60) defining an internal wall surface (36) and extending from the top surface (10) to the bottom surface (20)
**characterized in that** at least a portion of the internal wall surface (36) has a roughened surface topography (80) having, in combination, a macro average height from peak to valley of greater than 100 µm, a microtexture height from peak to valley of from about 0.5 to about 100 µm and a nanotexture height from peak to valley of from about 1 to about 1000 nm, wherein said roughened topography (80) facilitates attachment and proliferation of osteoblasts on the internal wall surface (36).

2. The spinal implant (1) of claim 1, wherein the body has at least one transverse aperture (70) extending at least partially along the transverse length of the body.

3. The spinal implant (1) of claim 1 or 2, wherein the body further comprises internal medial to lateral surfaces (35) having a roughened surface topography (80) having a microtexture height from peak to valley of from about 0.5 to about 100 µm and a nanotexture height from peak to valley of from about 1 to about 1000 nm.

4. The spinal implant (1) of any one of claims 1 to 3, wherein the body further comprises anterior to posterior passage surfaces (37) having a roughened surface topography (80) having a microtexture height from peak to valley of from about 0.5 to about 100 µm and a nanotexture height from peak to valley of from about 1 to about 1000 nm.

5. The spinal implant (1) of any one of claims 1 to 4, wherein the body comprises a substantially hollow center filled with bone graft materials.

## Patentansprüche

1. Zwischenwirbelimplantat (1), das einen Körper aus Titan oder Titanlegierung einschließt, wobei der Körper umfasst:
eine Oberseite (10);
eine Unterseite (20);
sich gegenüberliegende laterale Seiten (30);
sich gegenüberliegende anteriore (40) und posteriore (50) Abschnitte und mindestens eine vertikale Öffnung (60), die eine Innenwandoberfläche (36) definiert und sich von der Oberseite (10) zu der Unterseite (20) erstreckt;
**dadurch gekennzeichnet, dass** mindestens ein Abschnitt der Innenwandoberfläche (36) eine aufgeraute Oberflächentopographie (80) mit einer Kombination von einer durchschnittlichen Makrohöhe von der Spitze zum Tal von mehr als 100 µm, einer Mikrotexturhöhe von der Spitze zum Tal von etwa 0,5 bis etwa 100 µm und einer Nanotexturhöhe von der Spitze zum Tal von etwa 1 bis etwa 1000 nm aufweist, wobei die aufgeraute Topographie (80) Anhaftung und Proliferation von Osteoblasten an der Innenwandoberfläche (36) erleichtert.

2. Wirbelimplantat (1) nach Anspruch 1, wobei der Körper mindestens eine quer verlaufende Öffnung (70) aufweist, die sich mindestens teilweise entlang der quer verlaufenden Länge des Körpers erstreckt.

3. Wirbelimplantat (1) nach Anspruch 1 oder 2, wobei der Körper ferner innere mediale bis laterale Oberflächen (35) mit einer aufgerauten Oberflächentopographie (80) aufweist, die eine Mikrotexturhöhe von der Spitze zum Tal von etwa 0,5 bis etwa 100 µm und eine Nanotexturhöhe von der Spitze zum Tal von etwa 1 bis etwa 1000 nm aufweist.

4. Wirbelimplantat (1) nach einem der Ansprüche 1 bis 3, wobei der Körper ferner anteriore bis posteriode Durchgangsoberflächen (37) mit einer aufgerauten Oberflächentopographie (80) aufweist, die eine Mikrotexturhöhe von der Spitze zum Tal von etwa 0,5 bis etwa 100 µm und eine Nanotexturhöhe von der Spitze zum Tal von etwa 1 bis etwa 1000 nm aufweist.

5. Wirbelimplantat (1) nach einem der Ansprüche 1 bis 4, wobei der Körper eine im Wesentlichen hohle Mitte umfasst, die mit Knochenspanmaterialien gefüllt ist.

## Revendications

1. Implant spinal intervertébral (1) comprenant un corps en titane ou en alliage de titane, le corps comprenant :
une surface supérieure (10) ;
une surface inférieure (20) ;
des côtés latéraux opposés (30) ;
des parties antérieure (40) et postérieure (50) opposées ; et
au moins une ouverture verticale (60) délimitant une surface de paroi interne (36) et s'étendant de la surface supérieure (10) à la surface inférieure (20)
**caractérisé en ce qu'**au moins une partie de la surface de paroi interne (36) présente une topographie de surface rugueuse (80) ayant, en association, une macro-hauteur moyenne de crête à creux supérieure à 100 µm, une hauteur de microtexture de crête à creux d'environ 0,5 à environ 100 µm et une hauteur de nanotexture de crête à creux d'environ 1 à environ 1000 nm, dans lequel ladite topographie de surface rugueuse (80) facilite la fixation et la prolifération d'ostéoblastes sur la surface de paroi interne (36).

2. Implant spinal (1) selon la revendication 1, dans lequel le corps présente au moins une ouverture transversale (70) qui s'étend au moins partiellement le long de la longueur transversale du corps.

3. Implant spinal (1) selon la revendication 1 ou 2, dans lequel le corps comprend en outre des surfaces internes médiales à latérales (35) ayant une topographie de surface rugueuse (80) avec une hauteur de microtexture de crête à creux d'environ 0,5 à environ 100 µm et une hauteur de nanotexture de crête à creux d'environ 1 à environ 1000 nm.

4. Implant spinal (1) selon l'une quelconque des revendications 1 à 3, dans lequel le corps comprend en outre des surfaces de passage antérieures à postérieures (37) ayant une topographie de surface rugueuse (80) avec une hauteur de microtexture de crête à creux d'environ 0,5 à environ 100 µm et une hauteur de nanotexture de crête à creux d'environ 1 à environ 1000 nm.

5. Implant spinal (1) selon l'une quelconque des revendications 1 à 4, dans lequel le corps comprend un centre sensiblement creux rempli de matériau de greffe d'os.
